# EUROPÄISCHE PATENTANMELDUNG

(11) **EP 4 653 418 A1**
(43) Veröffentlichungstag der Anmeldung: **26.11.2025**
(21) Anmeldenummer: 24177225.0
(22) Anmeldetag: 22.05.2024
(51) Int. Cl.: C07C 201/08, C07C 265/14, C07C 263/10, C08G 18/10

(54) **VERFAHREN ZUR HERSTELLUNG VON ORGANISCHEM ISOCYANAT MIT VERBESSERTER NACHHALTIGKEIT**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Durch den Einsatz einer Vorrichtung zur Herstellung von organischem, aromatischem Isocyanat, mindestens enthaltend eine spezielle Produktionseinheit zur Bereitstellung von Salpetersäure und überhitztem Wasserdampf, eine Nitriereinheit, Hydriereinheit und Phosgeniereinheit, lässt sich eine verbesserte Nutzung nachhaltiger Energiequellen unter Verwertung von Abwärme als überhitzten Wasserdampf zur Wärmeenergieeinspeisung in weitere Verwertungsvorrichtungen, insbesondere in weitere Einheiten der Vorrichtung, erzielen. Teile dieser Vorrichtung werden wie in Anspruch 1 beschrieben durch einem Elektromotor angetrieben, um auf diese Weise die Ressourcen an Prozesswärme in Form von Wasserdampf effektiver über die gesamte Prozesskette der Vorrichtung verteilt zu nutzen, wodurch der Energieeinsatz nachhaltiger gestaltet wird.

## Beschreibung

Die Erfindung betrifft eine Vorrichtung zur Herstellung von organischem, aromatischem Isocyanat, mindestens enthaltend eine Produktionseinheit zur Bereitstellung von Salpetersäure und überhitztem Wasserdampf, eine Nitriereinheit, Hydriereinheit und Phosgeniereinheit, wobei die Vorrichtung eine verbesserte Nutzung nachhaltiger Energiequellen unter Verwertung von Abwärme als überhitzten Wasserdampf zur Wärmeenergieeinspeisung in weitere Verwertungsvorrichtungen, insbesondere in weitere Einheiten der Vorrichtung, erlaubt. Ebenso betrifft die Erfindung ein Verfahren zur Herstellung von organischem Isocyanat mit einer verbesserten Nutzung nachhaltiger Energiequellen und der aus dem Verfahren erhaltenen Abwärme als Rückenergie, sowie die Verwendung einer speziellen Produktionseinheit zur Bereitstellung von Salpetersäure und überhitztem Wasserdampf für die Versorgung, insbesondere der Nitriereinheit, Hydriereinheit und Phosgeniereinheit mit Wärmeenergie.

Wie aus dem Stand der Technik bekannt, wird organisches, aromatisches Isocyanat, wie z.B. Toluylendiisocyanat (TDI) oder Methylendi(phenylisocyanat) (MDI), üblicherweise durch Phosgenierung von aromatischem Amin, wie z.B. Toluylendiamin (TDA) oder Methylendianilin (MDA), mit Phosgen erhalten.

Das dafür benötigte aromatische Amin lässt sich über eine katalytische Hydrierung von organischem Nitroaromaten, wie z.B. Dinitrotoluol, mit Wasserstoff unter erhöhtem Druck an einem suspendierten, pulverförmigen Katalysator (z.B. Palladium auf Aktivkohle oder Raney-Nickel) darstellen. Hierbei wird ein Produktgemisch erhalten, welches neben dem gewünschten aromatischen Amin als Nebenbestandteil zusätzlich Wasser, sowie unerwünschte Isomere des aromatischen Amins, hoch- und niedrigsiedende Nebenprodukte und gegebenenfalls zugesetztes, organisches Lösemittel enthält. Solche Nebenbestandteile müssen vor der Zuführung des aromatischen Amins zur Phosgenierung abgetrennt werden.

Wie in der Druckschrift EP 0 757 034 A1 beschrieben, wird für die destillative Aufreinigung und Entwässerung (auch als Trocknung bezeichnet) des für die Phosgenierung benötigten organischen, aromatischen Amins, aber auch für die destillative Aufreinigung des organischen, aromatischen Isocyanates als Produkt, Wärmeenergie benötigt, die üblicherweise in Form von überhitztem Wasserdampf in das Verfahren eingebracht wird. Wie in der Druckschrift DE 19636191 A beschrieben, wird für die Aufkonzentrierung der für die Herstellung von organischen Nitroaromaten verwendeten Schwefelsäure gleichfalls überhitzter Wasserdampf eingesetzt. Überhitzter Wasserdampf weist bei gegebenem Druck definitionsgemäß eine Temperatur oberhalb der Siedetemperatur auf. Der überhitzte Wasserdampf wird in den meisten Verfahren des Standes der Technik zu einem Großteil durch Wasserdampferzeugung in einer Dampfkesselanlage mit Überhitzer unter Einsatz fossiler Brennstoffe wie Erdgas, Koks oder Erdölbasierten Brennstoffen, bereitgestellt. Die Bereitstellung des überhitzten Wasserdampfes stellt jedoch einen erheblichen Energieaufwand dar, der durch die Nutzung fossiler Energiequellen den CO₂-Ausstoß fördert und daher wenig nachhaltig ist.

Der in der katalytischen Reduktion als Ausgangsstoff eingesetzte Nitroaromat wird wiederum aus einer Nitrierung von organischer, aromatischer Verbindung, wie z.B. Toluol, mit Salpetersäure bereitgestellt.

Salpetersäure wird zunächst unter erhöhtem Druck an einem Katalysator (z.B. Platin, Platin-Rhodium) durch Umsetzung von Ammoniak NH₃ mit Sauerstoff, z.B. Luftsauerstoff, unter Oxidation gemäß Umsetzung (1) hergestellt:

(1) 4 NH₃ + 5 O₂ → 4 NO + 6 H₂O 907,3 kJ Reaktionswärme

In diesem Prozess entsteht neben Stockstoffmonoxid NO eine große Menge an Wärmeenergie. Das in diesem Prozess anfallende NO wird dann gemäß Umsetzung (2) zu Stickstoffdioxid NO₂ weiter aufoxidiert, wobei zusätzliche Wärme freigesetzt wird:

(2) 2 NO + O₂ → 2 NOz 113,1 kJ Reaktionswärme

Anschließend wird das auf diese Weise hergestellte Stickstoffdioxid NO₂ gemäß Umsetzung (3) in Wasser unter Erhalt von Salpetersäure und weiterer Wärme absorbiert:

(3) 4 NO₂ + O₂ + 2 H₂O → 4 HNO₃ 256,9 / 93,3 kJ Reaktionswärme

Die Absorption des NO₂ erfolgt wie die Oxidation des Ammoniaks bei erhöhtem Druck, vorzugsweise bei Drucken zwischen 4 bis 14 bar.

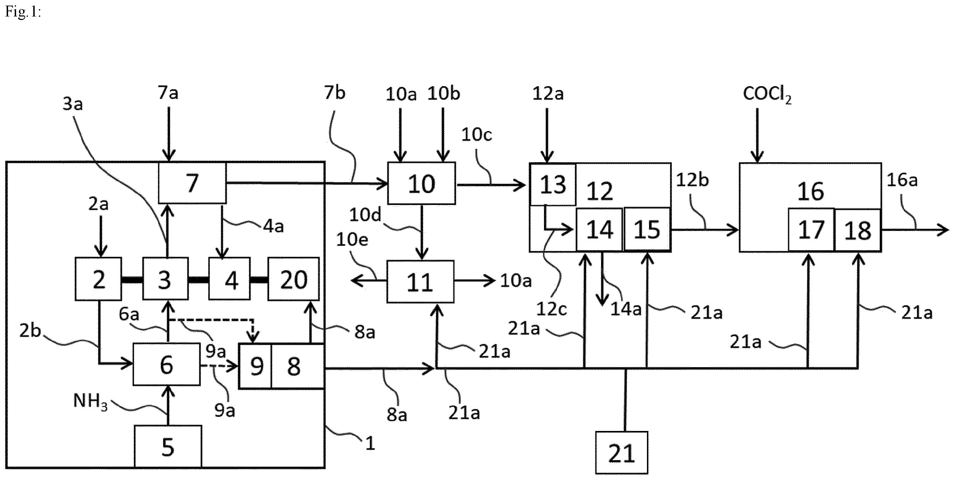

Die zur Durchführung des Gesamtprozesses der Salpetersäureherstellung notwendigen Drucke werden durch Kompression von beteiligtem Prozessgas mittels Kompressor erzeugt. Zu diesem Zweck wird beispielsweise der für die Ammoniakoxidation benötigte Sauerstoff üblicherweise als Luftsauerstoff in Form von komprimierter Prozessluft zugeführt. Die Sauerstoff-haltige Prozessluft wird dabei mittels Kompressor verdichtet und auf einen Druck gebracht, der sowohl der Oxidationsreaktion wie auch der späteren Absorptionsreaktion angepasst ist. Die für die Kompression notwendige Energie wird teilweise mittels Entspannung des aus der Absorption austretenden Restgases und ebenso durch die Verwertung der bei den obigen Umsetzungen (1) bis (3) freigesetzten Reaktionswärmen gewonnen. Aus diesen Wärmen wird Wasserdampf erzeugt, mit dem größtenteils die Turbine eines Turbokompressors zur Kompression der für die Salpetersäureherstellung genutzten Prozessgase betrieben wird. Ein geeigneter Kompressor wird beispielsweise in Fig. 1 der Druckschrift DE 10 2022 201 476 A1 beschrieben.

Eine Salpetersäure-Anlage kann nach dem Mono-Hochdruck-Verfahren betrieben werden. Bei diesem Verfahren erfolgt die Verbrennung des Ammoniaks und die Absorption der Stickstoffoxide bei etwa gleichem Druck von ca. 10 bar. Sind große Nenn-Kapazitäten und/oder höhere Säurekonzentrationen gefordert, kann eine Salpetersäure-Anlage nach dem Zwei-Druckverfahren betrieben werden. Beim Zwei-Druckverfahren geschieht die Verbrennung des eingesetzten Ammoniaks bei einem erhöhten Druck, der niedriger ist, als der bei der Absorption herrschende erhöhte Druck.

Für die Herstellung organischer Isocyanate können die dafür notwendigen organischen Ausgangsverbindungen, wie die organische Nitroverbindung oder das organische Amin (vgl. Druckschrift EP 0 757 034 A1), oder die Salpetersäure an den Produktionsstandort des organischen Isocyanates geliefert werden. Hierfür werden diese Ausgangsprodukte zunächst an einem dezentralen Standort produziert, der von dem Standort der zur Isocyanatproduktion genutzten Phosgenierungsvorrichtung meist derart weit entfernt ist, dass die genutzten Anlagen für einen Stofftransport nicht über eine Rohrleitung miteinander verbunden sein können. Der für diese Lieferungen notwendige, mit einem Transportmittel (wie Lastkraftwagen, Eisenbahn oder Schiff) durchzuführende Transport wirkt sich aufgrund der dafür aufzuwendenden Energie negativ auf die Nachhaltigkeit des Gesamtprozesses, i.e. des Prozesses der Herstellung im Gesamtverbund, aus.

Aufgabe der vorliegenden Erfindung war es daher, die Nachhaltigkeit der Herstellung von organischem Isocyanat im Gesamtverbund zu verbessern und insbesondere den CO₂-Fußabdruck des Herstellprozesses zu verringern. Hierfür sollte insbesondere der Energiebedarf und die dafür benötigte Energieform jedes Teilprozesses der Isocyanatherstellung bewertet und in die Optimierung der Effizienz und Nachhaltigkeit des Gesamtprozesses einbezogen werden.

Unter "Nachhaltigkeit" eines Verfahrens versteht der Fachmann in Anwendung der durch die UN geprägte Nachhaltigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre. Die Erfindung hat somit zur Aufgabe, die Produktion von organischen Isocyanaten, sowie daraus hergestelltem Polyurethan, nachhaltiger als die aus dem Stand der Technik bekannten Produktionsmethoden zu gestalten. Der Beitrag der Produktion von Isocyanat und Polyurethan zu einer sinkenden Befriedigung der Bedürfnisse zukünftiger Generationen soll vermindert bis vermieden werden.

Es wurde gefunden, dass sich insbesondere die Deckung des Energiebedarfs der Teilprozesse der Isocyanat-Herstellung nachhaltiger gestalten lässt, wenn die im Gesamtprozess anfallende Prozess-Wärme möglichst effizient und/oder vollständig in den Teilprozessen in Form von Dampf genutzt wird.

Ein erster Gegenstand der Erfindung ist somit eine Vorrichtung zur Herstellung von organischem, aromatischem Isocyanat, enthaltend:
mindestens eine Produktionseinheit zur Bereitstellung von Salpetersäure und Wasserdampf, wobei die Produktionseinheit enthält:
mindestens eine Verdichtungseinheit, enthaltend mindestens einen Kompressor zur Verdichtung von Sauerstoff-haltigem Gas und mindestens einen Auslass für das komprimierte, Sauerstoff-haltige Gas; und
mindestens eine Bereitstellungsvorrichtung zur Bereitstellung von Ammoniak; und
mindestens einen Oxidationsreaktor zur Oxidation des bereitgestellten Ammoniaks im Gemisch zumindest mit Sauerstoff aus dem komprimierten, Sauerstoff-haltigen Gas zu einem Stickstoffoxid-haltigen Produktgas unter Bereitstellung von Wärme; wobei der Oxidationsreaktor mindestens einen Auslass für Stickstoffoxid-haltiges Produktgas aufweist;
   und
mindestens eine Verdichtungseinheit, enthaltend mindestens einen Kompressor zur Verdichtung zumindest des Stickstoffoxids aus dem Produktgas und mindestens einen Auslass für komprimiertes, Stickstoffoxid-haltiges Produktgas
mindestens einen Absorptionsreaktor zur Umsetzung von zumindest Stickstoffoxid aus dem komprimierten Produktgas mit Wasser zu Salpetersäure; und
mindestens eine Dampfeinheit zur Bereitstellung von überhitztem Wasserdampf mit einer Temperatur von mindestens 200°C, wobei diese Dampfeinheit mindestens einen Wärmetauscher enthält, der zur Abführung von Wärme aus dem Oxidationsreaktor und/oder/ aus dem aus dem Oxidationsreaktor herausgeführten Produktgases, jeweils durch Übertragung der Wärme auf Wasser, zur Bereitstellung des überhitzten Wasserdampfes, konfiguriert ist;
   und
mindestens eine Nitriereinheit zur Nitrierung von organischer, aromatischer Verbindung zumindest unter Einsatz von in besagter Produktionseinheit hergestellter Salpetersäure und von Schwefelsäure unter Erhalt zumindest von organischer, aromatischer Nitroverbindung und abgereicherte Schwefelsäure, wobei diese Nitriereinheit mindestens eine Einheit zur Aufkonzentrierung der bei der Nitrierung anfallenden abgereicherten Schwefelsäure enthält und diese Nitriereinheit mit besagter Produktionseinheit zur Bereitstellung des Edukts Salpetersäure in Verbindung steht;
   und
mindestens eine Hydriereinheit zur Hydrierung von in der Nitriereinheit hergestellter organischer, aromatischer Nitroverbindung mit Wasserstoffgas unter Erhalt organischer, aromatischer Aminoverbindung, wobei die Hydriereinheit mindestens einen Hydrierreaktor, mindestens eine Einheit zur Entwässerung von Rohprodukt und mindestens eine Destillationseinheit enthält,
und wobei für die Bereitstellung der organischen, aromatischen Nitroverbindung als Edukt diese Hydriereinheit mit der Nitriereinheit in Verbindung steht;
   und
mindestens eine Phosgeniereinheit zur Phosgenierung von in der Hydriereinheit hergestellter, organischer, aromatischer Aminoverbindung unter Erhalt von organischer, aromatischer Isocyanatverbindung, wobei diese Phosgeniereinheit mindestens eine Einheit zur Phosgenentfernung und mindestens eine Destillationseinheit enthält,
und wobei diese Phosgeniereinheit zur Bereitstellung der organischen, aromatischen Aminoverbindung mit der Hydriereinheit in Verbindung steht;
wobei die mindestens eine Produktionseinheit zur Bereitstellung von Salpetersäure und Wasserdampf zusätzlich mindestens einen Elektromotor enthält, wobei die Kompressoren der besagten Verdichtungseinheiten der Produktionseinheit mit dem mindestens einen Elektromotor zum Antrieb verbunden sind.

Eine Verbindung von Vorrichtungsteilen ist wie üblich zur Gewährleistung eines Stoffstroms (eines Stoffstroms z.B. von Edukten oder von Zwischenprodukten) von einem Vorrichtungsteil zum anderen ausbildet. Diese Verbindung ist üblicherweise als Fluidverbindung ausgestaltet, welche als Teil einer Vorrichtung verstanden wird, der andere Vorrichtungsteile miteinander verbindet und durch den sich ein Stoff, der in jedem Aggregatzustand vorliegen kann, als Stoffstrom von einem Vorrichtungsteil zum nächsten transportieren lässt, beispielsweise eine Zuleitung in Form eines Rohres oder in Form anderer Vorrichtungsteile, durch die der Stoff hindurch transportiert wird. Der Begriff "in Verbindung stehen" bzw. "in Fluidverbindung stehen" bedeutet, dass benannte Vorrichtungsteile über eine solche Verbindung bzw. Fluidverbindung miteinander verbunden sind.

Die erfindungsgemäße Produktionseinheit zur Bereitstellung von Salpetersäure und Wasserdampf umfasst wie oben beschrieben jeweils mindestens eine der besagten Verdichtungseinheiten. Diese mindestens eine Verdichtungseinheit wird durch mindestens einen Elektromotor betrieben.

Der Einsatz eines Elektromotors für den Betrieb der mindestens einen Verdichtungsanlage ermöglicht es, den durch die Dampfeinheit erzeugten Dampf anderweitig zu nutzen als für den Antrieb der Verdichter-Wellen.

Es ist im Rahmen der vorliegenden Erfindung ganz besonders bevorzugt, wenn zur Bereitstellung von grünem, elektrischem Strom der Elektromotor mit einer Quelle für elektrischen Strom aus regenerativer Energie, insbesondere aus Windkraft, Wasserkraft oder Sonnenenergie, verbunden ist. Unter "regenerativer Energie" versteht der Fachmann Energie aus einer Energiequelle, die sich nicht erschöpft, wie z.B. Windenergie, Wasserenergie oder Sonnenenergie.

Es ist im Rahmen einer weiteren Ausführungsform erfindungsgemäß bevorzugt, wenn die besagten Verdichtungseinheiten zu einer Verdichtungseinheit zusammengefasst sind, welche zumindest die vorgenannten Kompressoren enthält, die mit mindestens einem Elektromotor zum Antrieb verbunden sind. Es ist somit erfindungsgemäß besonders bevorzugt, wenn die Produktionseinheit zur Bereitstellung von Salpetersäure und Wasserdampf als Verdichtungseinheit mindestens eine Verdichtungseinheit enthält, enthaltend
(i) mindestens einen Kompressor zur Verdichtung von Sauerstoff-haltigem Gas; und
(ii) mindestens einen Kompressor zur Verdichtung zumindest des Stickstoffoxids aus dem Produktgas; und
(iii) eine Antriebseinrichtung mit mindestens einem Elektromotor, die zumindest über Antriebswellen zum Antrieb des mindestens einem Kompressors zur Verdichtung von Sauerstoff-haltigem Gas und des mindestens einem Kompressors zur Verdichtung zumindest des Stickstoffoxids aus dem Produktgas verbunden ist; und
(iv) mindestens einen Auslass für das komprimierte, Sauerstoff-haltige Gas; und
(v) mindestens einen Auslass für komprimiertes, Stickstoffoxid-haltiges Produktgas; und gegebenenfalls zusätzlich
(vi) mindestens einen Gasexpander.

Aus dem komprimierten, Stickstoffoxid-haltigen Produktgas wird im Absorptionsreaktor der erfindungsgemäßen Vorrichtung nach der Absorption und gegebenenfalls einer Reinigung ein komprimiertes Restgas erhalten. Bei dem besagten Gasexpander (iv) der Verdichtungseinheit handelt es sich im Wesentlichen um eine Turbine, in der das unter Druck stehende, komprimierte Restgas expandiert und dabei Arbeit leistet. Zu diesem Zweck weist der Gasexpander einen Einlass für das komprimierte Restgas und einen Auslass für das expandierte, aber im Vergleich zum Atmosphärendruck weiterhin komprimierte Restgas auf. Dabei steht im Rahmen dieser Ausführungsform der besagte Einlass für das komprimierte Restgas mit einem in der Absorptionseinheit vorhandenem Auslass für das komprimierte Restgas in Verbindung.

Der Gasexpander kann zusätzlich die mit Kupplungen verbundene Antriebswelle antreiben. Der Gasexpander ist vorzugsweise als ein- oder mehrstufige Turbine ausgestaltet.

Es eignet sich ganz besonders bevorzugt eine mittels Elektromotor betriebene Variante der Verdichtungseinheit zu nutzen, die in der Druckschrift DE 10 2022 201 467 A1 oder EP 0 945 400 A2 beschrieben wird, jeweils mit den vorgesehenen Ein- und Auslässen.

Die Produktionseinheit zur Bereitstellung von Salpetersäure und Wasserdampf weist erfindungsgemäß mindestens eine Bereitstellungsvorrichtung zur Bereitstellung von Ammoniak auf. Eine solche Bereitstellungsvorrichtung kann beispielsweise ein Lagertank für verflüssigten Ammoniak sein, der zur Befüllung mit Ammoniak über einen Verdichter an eine Vorrichtung zur Produktion von Ammoniak verbunden ist. Ebenso kann der Lagertank beispielsweise mit entsprechendem flüssigem Ammoniak aus Transportbehältnissen wie z.B. Tankwagen, welche für die Lieferung zum Transport genutzt werden, befüllt sein. In jedem Fall ist es im Rahmen einer bevorzugten Ausführungsform vorteilhaft, solchen Ammoniak über die Bereitstellungsvorrichtung vorzuhalten, der Verfahrensprodukt eines Herstellverfahrens ist, in dem mittels Wasserelektrolyse erhaltener Wasserstoff (unter Einsatz von elektrischer Energie aus regenerativer Energiequalle) und Stickstoffgas aus der Luft (wie in der hiermit unter ausdrücklicher und vollumfänglicher Bezugnahme zitierten Druckschrift WO 2024/017890 A2 beschrieben) zu Ammoniak umgesetzt werden.

Die Produktionseinheit zur Bereitstellung von Salpetersäure und Wasserdampf weist erfindungsgemäß mindestens einen Oxidationsreaktor zur Oxidation des bereitgestellten Ammoniaks im Gemisch zumindest mit Sauerstoff aus dem in der entsprechenden Verdichtungseinheit komprimierten, Sauerstoff-haltigen Gas, wobei der Oxidationsreaktor mindestens einen Auslass für Stickstoffoxid-haltiges Produktgas aufweist. Solche im Rahmen der vorliegenden Erfindung nutzbaren Oxidationsreaktoren sind dem Fachmann beispielsweise aus den Druckschriften FR 2129012 A5, sowie Ullmann's Encyclopedia of Industrial Chemistry, Vol. 24, S. 177 (2012) bekannt, auf die hiermit ausdrücklich und vollinhaltlich Bezug genommen wird.

Für die Lösung der Aufgabe hat es sich als besonders effektiv erwiesen, wenn im Rahmen einer bevorzugten Ausführungsform der besagte Oxidationsreaktor der Vorrichtung derart ausgelegt ist, dass er im Betrieb zur Bereitstellung von mindestens 20 MW Wärmeenergie, bevorzugt mindestens 30 MW Wärmeenergie, fähig ist. Diese Auslegung des Oxidationsreaktors ist für den Fachmann durch Berechnung in Kenntnis der Prozesswärmen unter Variation der Skalierung des Reaktors, z.B. der Reaktorgröße oder bei kontinuierlichen Reaktoren des Durchsatzes, leicht umzusetzen und wird deshalb an dieser Stelle nicht näher beschrieben.

Die Produktionseinheit zur Bereitstellung von Salpetersäure und Wasserdampf weist erfindungsgemäß mindestens einen Absorptionsreaktor zur Umsetzung von zumindest Stickstoffoxid aus dem komprimierten Produktgas mit Wasser zu Salpetersäure auf. Solche im Rahmen der vorliegenden Erfindung nutzbaren Absorptionsreaktoren sind dem Fachmann beispielsweise aus den Druckschriften Ullmann's Encyclopedia of Industrial Chemistry, Vol. 24, S. 177 (2012) bekannt, auf die hiermit ausdrücklich und vollinhaltlich Bezug genommen wird.

Es ist erfindungsgemäß bevorzugt, wenn das besagte Stickstoffoxid ausgewählt ist aus mindestens einer Verbindung ausgewählt aus NO, NOz oder N₂O₄.

Die Produktionseinheit zur Bereitstellung von Salpetersäure und Wasserdampf weist erfindungsgemäß mindestens eine Dampfeinheit zur Bereitstellung von überhitztem Wasserdampf (bevorzugt zur Bereitstellung eines Stroms von überhitztem Wasserdampf) mit einer Temperatur von mindestens 200°C auf, wobei diese Dampfeinheit mindestens einen Wärmetauscher enthält, der zur Abführung von Wärme aus dem Oxidationsreaktor und/oder aus dem aus dem Oxidationsreaktor herausgeführten Produktgases, jeweils durch Übertragung der Wärme auf Wasser, zur Bereitstellung des überhitzten Wasserdampfes, konfiguriert ist. Solche im Rahmen der vorliegenden Erfindung nutzbaren Dampfeinheiten sind dem Fachmann dem Prinzip nach beispielsweise aus den Druckschriften "Waste heat boilers for nitric acid plants": Borsig Process Heat Exchanger GmbH 05/2023, EP 2 336 635B1 und EP 1 261 548 B2 bekannt, auf die hiermit ausdrücklich und vollinhaltlich Bezug genommen wird.

Im Rahmen einer bevorzugten Ausführungsform ist die besagte Dampfeinheit dazu ausgelegt, einen Wasserdampf-Strom von überhitztem Wasserdampf mit einem absoluten Druck von mindestens 6 bar (bevorzugt von mindestens 11 bar) und einer Temperatur von mindestens 200°C zu erzeugen. Dabei ist es ganz besonders bevorzugt, wenn die besagte Dampfeinheit dazu ausgelegt, einen Wasserdampf-Strom von überhitztem Wasserdampf mit einem absoluten Druck von mindestens 6 bar (bevorzugt von mindestens 11 bar) und mit einer Temperatur von mindestens 200°C zu erzeugen und der besagte Oxidationsreaktor der Vorrichtung derart ausgelegt ist, dass er im Betrieb zur Bereitstellung von mindestens 20 MW Wärmeenergie, bevorzugt mindestens 30 MW Wärmeenergie, für die Dampfeinheit fähig ist.

In der Produktionseinheit zur Bereitstellung von Salpetersäure und Wasserdampf bereitgestellter überhitzter Wasserdampf soll im Rahmen einer bevorzugten Ausführungsform der Erfindung in mindestens einer Verwertungsvorrichtung genutzt werden, die kein Bestandteil der Produktionseinheit zur Bereitstellung von Salpetersäure und Wasserdampf ist und damit als Verbraucher für den in der Dampfeinheit produzierten überhitzten Wasserdampf fungiert. Zu diesem Zweck steht mindestens ein Auslass der Dampfeinheit für überhitztem Wasserdampf in Verbindung mit der besagten Verwertungsvorrichtung. Als Verwertungvorrichtung eignet sich ganz besonders bevorzugt mindestens eine Destillationseinheit und/oder mindestens eine Entwässerungseinheit und/oder mindestens ein Wärmetauscher und/oder mindestens eine Dampf-Turbine.

Im Rahmen einer erfindungsgemäß besonders bevorzugten Ausführungsform der Vorrichtung enthält die Vorrichtung mindestens eine Verwertungsvorrichtung für den in der Dampfeinheit produzierten überhitzten Wasserdampf, wobei diese Verwertungsvorrichtung ein Teil einer Einheit, ausgewählt aus Nitriereinheit (einschließlich Schwefelsäure-Aufkonzentrierung), Hydriereinheit (einschließlich dazugehöriger Destillations-Stufe), Phosgeniereinheit (einschließlich dazugehöriger Destillations-Stufe) oder einer Kombination aus mindestens zwei dieser Einheiten, ist. Zu diesem Zweck ist zur Verwertung des überhitzten Wasserdampfes mindestens eine Verwertungsvorrichtung von mindestens einer Einheit, ausgewählt aus mindestens einer Einheit der Gruppe, die gebildet wird aus Nitriereinheit (einschließlich Schwefelsäure-Aufkonzentrierung), Hydriereinheit und Phosgeniereinheit mit mindestens einem Auslass für überhitztem Wasserdampf der Dampfeinheit zur Bereitstellung des überhitzten Wasserdampfes verbunden. Im Rahmen einer ganz besonders bevorzugten Ausführungsform der Erfindung, in der mindestens eine Verwertungsvorrichtung, ausgewählt aus Destillationseinheit, Entwässerungseinheit, Wärmetauscher oder Dampf-Turbine, die Teil mindestens einer Einheit, ausgewählt aus Nitriereinheit (einschließlich Schwefelsäure-Aufkonzentrierung), Hydriereinheit oder Phosgeniereinheit, mit mindestens einem Auslass für überhitztem Wasserdampf der Dampfeinheit zur Bereitstellung des überhitztem Wasserdampfs verbunden. In dieser Ausführungsform fungieren somit die Destillationseinheit, die Entwässerungseinheit, die Wärmetauscher oder die Dampf-Turbinen aus vorhandenen Anlagenteilen der Nitriereinheit und/oder der Hydriereinheit und/oder der Phosgeniereinheit als Verwertungsvorrichtung.

Im Rahmen einer weiteren, ganz besonders bevorzugten Ausführungsform soll die Nitriereinheit (einschließlich der Schwefelsäure-Aufkonzentrierung), Hydriereinheit (einschließlich dazugehöriger Entwässerung und/oder Destillations-Stufe) und Phosgeniereinheit (einschließlich dazugehöriger Destillations-Stufe) in Summe ihrer Verwertungsvorrichtungen zur Nutzung von mindestens 25 % der Gesamtmenge des in der Produktionseinheit zur Herstellung von Salpetersäure herstellbaren, überhitzten Wasserdampfes ausgelegt sein und entsprechend einer vorgesehenen Wasserdampf-Nutzung mit mindestens einem Auslass für überhitztem Wasserdampf der Dampfeinheit in Verbindung stehen.

Es ist bekannt, dass bei der Nitrierung organischer, aromatischer Verbindungen (wie z.B. Benzol, Toluol, Xylol, insbesondere von Benzol zu Nitrobenzol) mit Nitriersäure, einem Gemisch von Schwefelsäure (z.B. Oleum und/oder Schwefelsäure verschiedener Konzentration) und Salpetersäure, als zwangsläufiges Nebenprodukt eine durch das Reaktionswasser abgereicherte Schwefelsäure anfällt. Die erfindungsgemäße Vorrichtung enthält mindestens eine Nitriereinheit zur Nitrierung von organischer, aromatischer Verbindung zumindest unter Einsatz von in besagter Produktionseinheit hergestellter Salpetersäure und von Schwefelsäure unter Erhalt zumindest von organischer, aromatischer Nitroverbindung und abgereicherter Schwefelsäure, wobei diese Nitriereinheit mit besagter Produktionseinheit zur Bereitstellung des Edukts Salpetersäure in Verbindung steht und mit einer Aufkonzentrierung der abgereicherten Schwefelsäure ausgestattet ist. Solche im Rahmen der vorliegenden Erfindung nutzbaren Nitriereinheiten mit Einheiten zur Schwefelsäure-Aufkonzentrierung sind dem Fachmann beispielsweise aus den Druckschriften EP 1 508 563 A oder Ullmanns Enzyklopädie der technischen Chemie, 4. Auflage, Band 17, S. 392, DE 19636191 A und US 2,256,999 A bekannt, auf die hiermit ausdrücklich und vollinhaltlich Bezug genommen wird. Im Allgemeinen beträgt der Gehalt der erhaltenen abgereicherten Schwefelsäure etwa zwischen 20 und 96 Gew.-%, insbesondere etwa 60 bis 80 Gew.-% H₂SO₄, je nach Konzentration der in der Nitriersäure eingesetzten Schwefelsäure und der bei der Nitrierung pro Gewichtseinheit Nitriersäure anfallenden Menge Reaktionswasser, das von der Schwefelsäure aufgenommen wird.

Im Rahmen einer bevorzugten Ausführungsform ist die besagte Nitriereinheit (d.h. inklusive der Schwefelsäure-Aufkonzentrierung) zur Nutzung von mindestens 25 % der Gesamtmenge der in der Produktionseinheit bereitgestellten Salpetersäure ausgelegt und steht mit mindestens einem Auslass für Salpetersäure des Absorptionsreaktors in Verbindung. Von dieser Ausführungsform wird zur Steigerung der Nachhaltigkeit weiter bevorzugt eine Variante ausgeführt, in der der besagte Oxidationsreaktor der besagten Produktionseinheit zur Bereitstellung von Salpetersäure und Wasserdampf derart ausgelegt ist, dass er im Betrieb zur Bereitstellung von mindestens 20 MW Wärmeenergie, bevorzugt mindestens 30 MW Wärmeenergie, fähig ist.

Die erfindungsgemäße Vorrichtung enthält mindestens eine Hydriereinheit zur Hydrierung von in der Nitriereinheit hergestellter organischer, aromatischer Nitroverbindung mit Wasserstoffgas unter Erhalt von organischer, aromatischer Aminoverbindung, wobei die Hydriereinheit mindestens einen Hydrierreaktor, mindestens eine Einheit zur Entwässerung von Rohprodukt und mindestens eine Destillationseinheit enthält und wobei für die Bereitstellung der organischen, aromatischen Nitroverbindung als Edukt diese Hydriereinheit mit der Nitriereinheit in Verbindung steht.

Die Entwässerung des Hydrier-Rohrproduktes ist sehr energieintensiv und wird in der Regel einstufig oder mehrstufig mit Heizdampf und ggf. unter Vakuum durchgeführt. Die zum Einsatz kommenden Wärmetauscher können dabei Fallfilmverdampfer, Einsteckverdampfer, Kessel-Verdampfer (Kettle-type Reboilers), Rohrbündelwärmetauscher oder beliebige andere dem Fachmann bekannte Wärmetauscher-Designs sein. Die Abtrennung des Wassers findet in einer oder mehreren Destillationskolonnen statt, die bevorzugt mit Böden oder strukturierten Packungen ausgestattet sind. Im Rahmen einer weiteren bevorzugten Ausführungsform der erfindungsgemäßen Vorrichtung steht zur Versorgung mit Wasserdampf die besagte mindestens eine Einheit zur Entwässerung des Rohproduktes mit mindestens einem Auslass für überhitztem Wasserdampf der Dampfeinheit in Verbindung.

Die Aufreinigung einer aromatischen Aminoverbindung findet in der Regel in einstufigen oder mehrstufigen Destillationseinheiten zur Abtrennung verbleibenden Wassers, von Leichtsiedern und/ oder von hochsiedenden Nebenprodukten sowie ggf. von unerwünschten Amin-Isomeren statt, wobei in der Regel Rektifikations-Kolonnen mit Böden, strukturierten Packungen oder Füllkörpern zum Einsatz kommen. Die Rektifikation kann hierbei unter Vakuum stattfinden. Die zum Einsatz kommenden Wärmetauscher können dabei Fallfilmverdampfer, Einsteckverdampfer, Kessel-Verdampfer (Kettle-type Reboilers), Rohrbündelwärmetauscher oder beliebige andere dem Fachmann bekannte Wärmetauscher-Designs sein. Im Rahmen einer weiteren bevorzugten Ausführungsform der erfindungsgemäßen Vorrichtung steht zur Versorgung mit Wasserdampf mindestens eine Destillationseinheit der Hydriereinheit mit mindestens einem Auslass für überhitztem Wasserdampf der Dampfeinheit in Verbindung.

Solche im Rahmen der vorliegenden Erfindung nutzbaren Hydriereinheiten einschließlich nachgeschalteter Destillations- und Entwässerungs-Stufen sind dem Fachmann beispielsweise aus den Druckschriften EP 2 263 997 A, EP 0 784 505 A, EP 1 602 640 A und EP 1 746 083 A bekannt, auf die hiermit ausdrücklich und vollinhaltlich Bezug genommen wird.

Die erfindungsgemäße Vorrichtung enthält mindestens eine Phosgeniereinheit zur Phosgenierung von in der Hydriereinheit hergestellter, organischer, aromatischer Aminoverbindung unter Erhalt von organischer, aromatischer Isocyanatverbindung, wobei diese Phosgeniereinheit mindestens eine Einheit zur Phosgenentfernung und mindestens eine Destillationseinheit enthält, wobei diese Phosgeniereinheit mit besagter Hydriereinheit zur Bereitstellung der organischen, aromatischen Aminoverbindung als Edukt in Verbindung steht. Solche im Rahmen der vorliegenden Erfindung nutzbaren Phosgeniereinheiten einschließlich nachgeschalteter Destillations-Stufe sind dem Fachmann beispielsweise aus den Druckschriften Six, Christian & Richter, Frank. (2003). Isocyanates, Organic: Ullmann's Encyclopedia of Industrial Chemistry, Vol.20, S.63-82 (https://doi.org/10.1002/14356007.a14_611), EP 1 371 636 A, WO 2011/003532 A1 und EP 1 371 635 A bekannt, auf die hiermit ausdrücklich und vollinhaltlich Bezug genommen wird.

In den für die Aufarbeitung organischer Isocyanate im Stand der Technik beschriebenen Destillations- und Aufarbeitungs-Verfahren wird Heizdampf zur Verdampfung und zum Vorheizen von Isocyanat, Lösemittel oder Phosgen eingesetzt. Die verwendeten Wärmetauscher können dabei Fallfilmverdampfer, Einsteckverdampfer, Kessel-Verdampfer (Kettle-type Reboilers), Rohrbündelwärmetauscher oder beliebige andere dem Fachmann bekannte Wärmetauscher-Designs sein. Die Destillationsverfahren können unter Vakuum durchgeführt werden. Die destillative Entfernung von Lösemittel, Leichsiedern und/ oder hochsiedenden Nebenprodukten findet in einer ein- mehrstufigen Destillation, ggf. unter Vakuum statt. Zum Einsatz kommen in der Regel Rektifikationskolonnen mit Böden, strukturierten Packungen oder Füllkörper-Schüttungen. Im Rahmen einer weiteren bevorzugten Ausführungsform der erfindungsgemäßen Vorrichtung steht zur Versorgung mit Wasserdampf mindestens eine Destillationseinheit der Phosgeniereinheit mit mindestens einem Auslass für überhitztem Wasserdampf der Dampfeinheit in Verbindung.

Die Vorrichtung des ersten Gegenstandes der Erfindung sowie deren Ausführungsformen eignen sich jeweils für die Durchführung des erfindungsgemäßen Verfahrens zur Isocyanatherstellung. Ein weiterer Gegenstand der Erfindung ist daher ein Verfahren zur Herstellung von organischem, aromatischem Isocyanat, enthaltend zumindest die folgenden Schritte:
Herstellung von Salpetersäure und überhitztem Wasserdampf mit einer Temperatur von mindestens 200°C durch zumindest folgende Schritte:
   Verdichtung eines Sauerstoff-haltigen Gases zu komprimiertem, Sauerstoff-haltigem Gas unter Einsatz mindestens eines Kompressors einer Verdichtungseinheit, der zumindest durch mindestens einen Elektromotor angetrieben wird;
   Vermischen von Ammoniak mit dem komprimierten, Sauerstoff-haltigen Gas und anschließender Oxidation des Ammoniaks unter Erhalt von Stickstoffoxid-haltigem Produktgas und Wärme;
   Umwandlung der Wärme aus obiger Oxidation unter Einsatz mindestens eines Wärmetauschers zu einem Strom von überhitztem Wasserdampf mit einer Temperatur von mindestens 200°C;
   Verdichtung des Stickstoffoxid-haltigen Produktgases aus der Oxidation unter Einsatz mindestens eines weiteren Kompressors einer weiteren Verdichtungseinheit, der durch einen Elektromotor angetrieben wird;
   Umsetzung von Stickstoffoxid aus dem komprimierten Stickstoffoxid-haltigem Produktgas mit Wasser zu Salpetersäure;
   Transport des überhitzten Wasserdampfes durch eine Transporteinheit zu mindestens einer Einheit zur Nutzung des Wasserdampf-Stromes;
Herstellung von organischer, aromatischer Nitroverbindung durch Umsetzung von organischer, aromatischer Verbindung mit zumindest der zuvor hergestellten Salpetersäure und mit Schwefelsäure;
Hydrierung der organischen, aromatischen Nitroverbindung mit Wasserstoffgas zu organischer, aromatischer Aminoverbindung und Aufarbeitung des Rohproduktes durch zumindest Destillation;
Phosgenierung der organischen, aromatischen Aminoverbindung mit Phosgen zu organischer, aromatischer Isocyanatverbindung und Aufarbeitung des Rohproduktes durch zumindest Destillation;
mit der Maßgabe, dass die Einheit zur Nutzung des Wasserdampf-Stromes von den zuvor genannten Einheiten zur Verdichtung von Sauerstoff-haltigem Gas und zur Verdichtung von Stickstoffoxid-haltigem Gas verschieden ist.

Für eine erhöhte Bereitstellung von Wasserdampf in Form des Wasserdampfstromes, wird im Rahmen des erfindungsgemäßen Verfahrens der besagte mindestens eine Kompressor einer Verdichtungseinheit durch mindestens einen Elektromotor angetrieben. Dabei ist es im Rahmen einer bevorzugten Ausführungsform vorteilhaft, wenn die besagten Verdichtungseinheiten zumindest durch mindestens einen Elektromotor angetrieben werden, der mit elektrischem Strom aus erneuerbarer Energie, insbesondere aus Windkraft, Wasserkraft oder Sonnenenergie, betrieben wird.

Eine weitere bevorzugte Ausführungsform des Verfahrens gekennzeichnet sich dadurch, dass die besagten Verdichtungseinheiten zumindest durch denselben Elektromotor angetrieben werden.

Für die Bereitstellung des Wasserdampf-Stromes hat es sich als vorteilhaft herausgestellt, wenn in einer weiteren bevorzugten Ausführungsform des Verfahrens die besagte bei der Oxidation des Ammoniaks bereitgestellte Wärme eine Wärmeenergie von mindestens 20 MW, bevorzugt von mindestens 30 MW besitzt.

Für den Transport und die Verwertung des im Verfahren erzeugten Wasserdampf-Stromes hat es sich als vorteilhaft herausgestellt, wenn in einer weiteren bevorzugten Ausführungsform des Verfahrens der besagte Wasserdampf-Strom einen absoluten Druck von mindestens 6 bar, bevorzugt von mindestens 11 bar, aufweist.

Das besagte Stickstoffoxid des Stickstoffoxid-haltigen Produktgases wird bevorzugt ausgewählt aus mindestens einer Verbindung ausgewählt aus NO, NO₂ oder N₂O₄.

Der Strom des überhitzten Wasserdampfes soll im Rahmen einer bevorzugten Ausführungsform des Verfahrens in mindestens einer Einheit zur Nutzung des Wasserdampf-Stromes genutzt werden, die von den zuvor genannten Einheiten zur Verdichtung von Sauerstoff-haltigem Gas und zur Verdichtung von Stickstoffoxid-haltigem Gas verschieden ist und ausgewählt wird aus mindestens einer Einheit ausgewählt aus Destillationseinheit, Entwässerungseinheit, Wärmetauscher, Dampf-Turbine.

Im Rahmen einer bevorzugten Ausführungsform des Verfahrens wird das Rohprodukt aus der Herstellung von organischer, aromatischer Nitroverbindung zusätzlich durch Destillation in einer Destillationseinheit aufgearbeitet und Wasserdampf aus dem besagten transportierten Wasserdampf-Strom dem Betrieb der Destillationseinheit zugeführt. Beispiele für entsprechend geeignete Destillationseinheiten wurden zur Beschreibung der erfindungsgemäßen Vorrichtung des ersten Erfindungsgegenstandes genannt.

Das erfindungsgemäße Verfahren enthält im Rahmen einer weiteren besonders bevorzugten Ausführungsform zusätzlich mindestens einen Schritt zur Aufkonzentrierung der bei der Umsetzung von organischer, aromatischer Verbindung mit zumindest der zuvor hergestellten Salpetersäure und mit Schwefelsäure als Nebenprodukt anfallenden abgereicherten Schwefelsäure.

Mindestens ein Schritt, ausgewählt aus mindestens einem der vorgenannten zusätzlichen Schritte und/oder mindestens einem der im Verfahren zwingend vorgesehenen Destillationsschritte, kann durch Betreiben einer Verwertungsvorrichtung unter Nutzung des Wasserdampf-Stroms (insbesondere mindestens einer Verwertungsvorrichtung ausgewählt aus Wärmetauscher und/oder Dampf-Turbine) durchgeführt werden.

Für die Aufkonzentrierung der bei der Umsetzung von organischer, aromatischer Verbindung mit zumindest der zuvor hergestellten Salpetersäure und mit Schwefelsäure als Nebenprodukt anfallenden abgereicherten Schwefelsäure kann Wasser in einer Destillationseinheit unter Einsatz des Wasserdampf-Stromes als Wärmequelle über einen Wärmetauscher der Destillationseinheit aus der abgereicherten Schwefelsäure entfernt werden.

Für die Destillation der organischen, aromatischen Aminoverbindung gilt das für die Destillation des Rohproduktes aus der Herstellung von organischer, aromatischer Aminoverbindung im Rahmen der ersten Erfindungsgegenstandes Gesagte *mutatis mutandis.*

Im Rahmen der Aufarbeitung des Rohproduktes bei der Phosgenierung ist es erfindungsgemäß bevorzugt, zunächst überschüssiges Phosgen aus dem Rohprodukt zu entfernen (beispielsweise durch) und danach zumindest die Destillation der organischen, aromatischen Isocyanatverbindung durchzuführen. Es gilt das im Rahmen der ersten Erfindungsgegenstandes entsprechend für die Destillation des Rohproduktes aus der Phosgenierung Gesagte.

Im Rahmen einer weiteren besonders bevorzugten Ausführungsform des Verfahrens wird dieses in einer Vorrichtung des ersten Erfindungsgegenstandes bzw. deren Ausführungsformen ausgeführt.

Ein dritter Gegenstand der Erfindung ist die Verwendung der mindestens einen Produktionseinheit für Salpetersäure und überhitztem Wasserdampf in einer Vorrichtung des ersten Erfindungsgegenstandes zur Versorgung von mindestens einer Einheit, ausgewählt aus der Gruppe, die gebildet wird aus Nitriereinheit, Hydriereinheit und Phosgeniereinheit, mit überhitztem Wasserdampf mit einer Temperatur von mindestens 200°C zur Abdeckung von mindestens 25% des Bedarfes dieser besagten Einheiten an Heizdampf mit einem absoluten Druck von mehr als 5 bar, insbesondere von mindestens 6 bar, weiter bevorzugt von mindestens 11 bar .

Anhand folgender Beispiele wird die Erfindung illustriert, ohne die Erfindung auf den Gegenstand dieser Beispiele zu beschränken.

### Beispiele

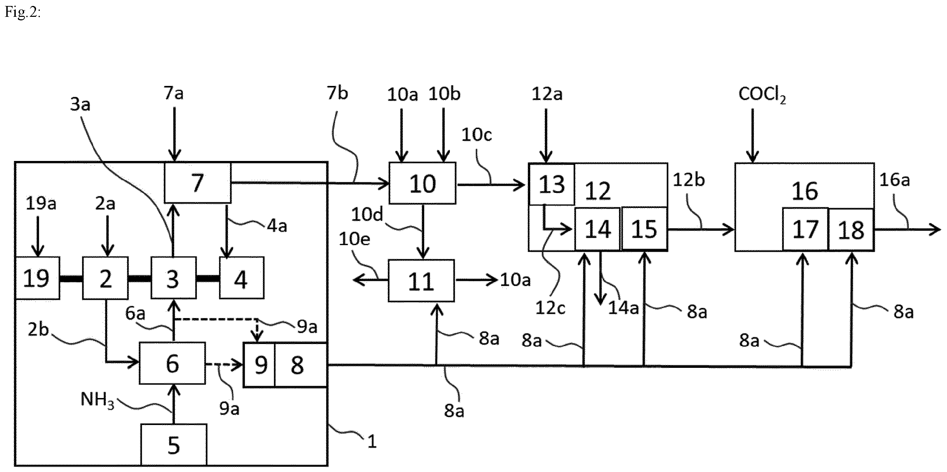

### Legende zu den Figuren Fig.1 und Fig.2:

- 1: Produktionseinheit zur Bereitstellung von Salpetersäure 7b und Wasserdampf 8a
- 2: Verdichtungseinheit mit Kompressor zur Verdichtung von Sauerstoff-haltigem Gas
- 2a: Sauerstoff-haltiges Edukt-Gas (z.B. Luft)
- 2b: komprimiertes, Sauerstoff-haltiges Edukt-Gas (z.B. komprimierte Luft)
- 3: Verdichtungseinheit mit Kompressor zur Verdichtung von Stickstoffoxid-haltigem Produktgas
- 3a: komprimiertes, Stickstoffoxid-haltiges Produktgas
- 4: Gasexpander für komprimiertes Restgas (4a)
- 4a: komprimiertes Restgas
- 5: Bereitstellungsvorrichtung zur Bereitstellung von Ammoniak
- 6: Oxidationsreaktor
- 6a: Stickstoffoxid-haltiges Produktgas
- 7: Absorptionsreaktor
- 7a: Wasser
- 7b: Salpetersäure
- 8: Dampfeinheit
- 8a: überhitzter Wasserdampf mit einer Temperatur von mindestens 200°C
- 9: Wärmetauscher
- 9a: Wärme
- 10: Nitriereinheit
- 10a: Schwefelsäure
- 10b: organische, aromatische Verbindung (z.B. Benzol, Toluol)
- 10c: organische, aromatische Nitroverbindung
- 10d: abgereicherte Schwefelsäure
- 10e: abgetrenntes Wasser
- 11: Einheit zur Aufkonzentrierung
- 12: Hydriereinheit
- 12a: Wasserstoffgas
- 12b: organische, aromatische Aminoverbindung
- 12c: Rohprodukt
- 13: Hydrierreaktor
- 14: Einheit zur Entwässerung
- 14a: abgetrenntes Wasser
- 15: Destillationseinheit
- 16: Phosgeniereinheit
- 16a: organische, aromatische Isocyanatverbindung (z.B. TDI, MDI)
- 17: Einheit zur Phosgenentfernung
- 18: Destillationseinheit
- 19: Elektromotor
- 19a: elektrischer Strom aus regenerativer Energie
- 20: Dampfturbine
- 21: Dampferzeugung unter Einsatz fossiler Brennstoffe
- 21a: Dampf aus Dampferzeugung 21 und Dampfeinheit 8

In den Figuren Fig. 1 und Fig.2 symbolisiert die durch die dicke Linie dargestellte Verbindung zwischen den einzelnen Verdichtereinheiten 2, 3 , dem Expander 4 und dem Elektromotor 19 jeweils eine für den Antrieb der Verdichtereinheiten vorgesehene Verbindung mit entsprechenden Kupplungsvorrichtungen. Weitere Linien zwischen den einzelnen Komponenten der Vorrichtung zur Herstellung von organischem, aromatischem Isocyanat stellen Verbindungen dar, die zur Gewährleistung eines Stoffstroms (eines Stoffstroms z.B. von Edukten oder von Zwischenprodukten) von einem Vorrichtungsteil zum anderen ausgebildet sind. Diese Verbindungen sind als Fluidverbindung ausgestaltet durch den sich der entsprechende für die Verbindung vorgesehene Stoff (siehe jeweilige Kennzeichnung) als Stoffstrom von einem Vorrichtungsteil zum nächsten transportieren lässt, beispielsweise eine Zuleitung in Form eines Rohres oder in Form anderer Vorrichtungsteile, durch die der Stoff hindurch transportiert wird. Die Fließrichtung wird durch eine Pfeilspitze markiert.

In Fig. 1 und Fig.2 wird jeweils eine Vorrichtung zur Herstellung von organischer, aromatischer Isocyanatverbindung gezeigt, enthaltend :
eine Produktionseinheit **1** zur Bereitstellung von Salpetersäure **7b** und Wasserdampf **8a,** wobei die Produktionseinheit **1** enthält:
   eine Verdichtungseinheit **2,** enthaltend mindestens einen Kompressor zur Verdichtung von Sauerstoff-haltigem Gas **2a** und mindestens einen Auslass für das komprimierte, Sauerstoff-haltige Gas **2b;** und
   eine Bereitstellungsvorrichtung **5** zur Bereitstellung von Ammoniak; und
   einen Oxidationsreaktor **6** zur Oxidation des bereitgestellten Ammoniaks im Gemisch zumindest mit Sauerstoff aus dem komprimierten, Sauerstoff-haltigen Gas **2b** zu einem Stickstoffoxid-haltigen Produktgas **6a** unter Bereitstellung von Wärme **9a;** wobei der Oxidationsreaktor **6** mindestens einen Auslass für Stickstoffoxid-haltiges Produktgas **6a** aufweist; und
   eine Verdichtungseinheit **3,** enthaltend mindestens einen Kompressor zur Verdichtung zumindest des Stickstoffoxids aus dem Produktgas **6a** und mindestens einen Auslass für komprimiertes, Stickstoffoxid-haltiges Produktgas **3a;**
   einen Absorptionsreaktor **7** zur Umsetzung von zumindest Stickstoffoxid aus dem komprimierten Produktgas **3a** mit Wasser **7a** zu Salpetersäure **7b;** und
   eine Dampfeinheit **8** zur Bereitstellung von überhitztem Wasserdampf **8a** mit einer Temperatur von mindestens 200°C, wobei diese Dampfeinheit **8** mindestens einen Wärmetauscher **9** enthält, der zur Abführung von Wärme **9a** aus dem Oxidationsreaktor **6** und/oder/ aus dem aus dem Oxidationsreaktor herausgeführten Produktgases **6a,** jeweils durch Übertragung der Wärme **9a** auf Wasser, zur Bereitstellung des überhitzten Wasserdampfes **8a,** konfiguriert ist;
   und
eine Nitriereinheit **10** zur Nitrierung von organischer, aromatischer Verbindung **10a** zumindest unter Einsatz von in besagter Produktionseinheit hergestellter Salpetersäure **7b** und von Schwefelsäure **10b** unter Erhalt von zumindest organischer, aromatischer Nitroverbindung **10c** und abgereicherter Schwefelsäure **10d,** wobei diese Nitriereinheit **10** mindestens eine Einheit zur Aufkonzentrierung **11** der bei der Nitrierung anfallenden abgereicherten Schwefelsäure **10d** und einen Auslass für das in der Aufkonzentrierung **11** abgetrennte Wasser **10e** enthält und diese Nitriereinheit **10** mit besagter Produktionseinheit **1** zur Bereitstellung des Edukts Salpetersäure **7b** in Verbindung steht;
   und
eine Hydriereinheit **12** zur Hydrierung von in der Nitriereinheit hergestellter organischer, aromatischer Nitroverbindung **10c** mit Wasserstoffgas **12a** unter Erhalt organischer, aromatischer Aminoverbindung **12b,** wobei die Hydriereinheit **12** mindestens einen Hydrierreaktor **13,** mindestens eine Einheit **14** zur Entwässerung von Rohprodukt, einen Auslass für das in der Einheit **14** abgetrennte Wasser **14a** und mindestens eine Destillationseinheit **15** enthält,
und wobei für die Bereitstellung der organischen, aromatischen Nitroverbindung **10c** als Edukt diese Hydriereinheit **12** mit der Nitriereinheit **10** in Verbindung steht;
   und
eine Phosgeniereinheit **16** zur Phosgenierung von in der Hydriereinheit **12** hergestellter, organischer, aromatischer Aminoverbindung **12b** unter Erhalt von organischer, aromatischer Isocyanatverbindung **16a,** wobei diese Phosgeniereinheit **16** mindestens eine Einheit zur Phosgenentfernung **17** und mindestens eine Destillationseinheit **18** enthält,
und wobei diese Phosgeniereinheit **16** zur Bereitstellung der organischen, aromatischen Aminoverbindung **12b** mit der Hydriereinheit **12** in Verbindung steht.

Gemäß Fig.2 enthält die erfindungsgemäße Ausführungsform der Vorrichtung eine Produktionseinheit **1** zur Bereitstellung von Salpetersäure **7b** und Wasserdampf **8a** zusätzlich einen mit elektrischem Strom aus regenerativer Energie **19a** betriebenen Elektromotor **19,** wobei die Kompressoren der besagten Verdichtungseinheiten **2, 3** der Produktionseinheit **1** mit dem Elektromotor **19** zum Antrieb verbunden sind. Weiterhin enthält die Produktionseinheit **1** der Fig.2 zusätzlich einen Expander **4** für komprimiertes Restgas **4a** aus der Absorptionsvorrichtung **7.** Der Gasexpander **4** trägt über seine Gasturbine gleichfalls zum Antrieb der Verdichtungseinheiten **2, 3** bei.

Gemäß Fig. 1 enthält die nicht erfindungsgemäße Ausführungsform der Vorrichtung eine Produktionseinheit **1** zur Bereitstellung von Salpetersäure **7b** und Wasserdampf **8a** zusätzlich eine mit dem Wasserdampf **8a** betriebene Dampfturbine **20,** wobei die Kompressoren der besagten Verdichtungseinheiten **2, 3** der Produktionseinheit **1** mit der Dampfturbine **20** zum Antrieb verbunden sind. Weiterhin enthält die Produktionseinheit **1** der Fig. 1 zusätzlich einen Expander **4** für komprimiertes Restgas **4a** aus der Absorptionsvorrichtung **7.** Der Gasexpander **4** trägt über seine Gasturbine gleichfalls zum Antrieb der Verdichtungseinheiten **2, 3** bei. Gemäß Fig. 1 wird der restliche in der Produktionseinheit **1** erzeugte Wasserdampf **8a** gemeinsam mit in einer Dampferzeugung **21** unter Einsatz fossiler Brennstoffe erzeugtem, überhitztem Wasserdampf in den Verwertungseinheiten **11, 14, 15, 17, 18** genutzt.

Nachfolgende Beispiele erfolgen unter Nutzung der jeweiligen Vorrichtungen:

### Beispiel 1 (Vorrichtung gemäß Fig.1, nicht erfindungsgemäß):

Die in Fig. 1 gezeigte Vorrichtung zur Herstellung von organischem, aromatischen Isocyanat **16a** umfasst eine Produktionseinheit **1** zur Bereitstellung von Salpetersäure **7b** und Wasserdampf **8a,** welche 70 t/h 68%ige Salpetersäure produziert, wobei die Abwärme des Oxidationsreaktors **6** in Form von 48 t/h hochgespannten Dampf **8a** mit einem absoluten Druck von 46 bar anfällt. Von diesen erzeugten 48 t/h Dampf **8a** (abs. Druck 46 bar) werden 30 t/h für die interne Dampf-Turbine **20** zum Antrieb der Verdichtungseinheiten **2, 3** eingesetzt (entspricht 6,3 MW Antriebsleistung) und 18 t/h für die weitere Dampf-Nutzung der Einheiten **10, 12** und **16** bereitgestellt.

Die Gesamtheit der produzierten Salpetersäure **7b** (70 t/h) ist derart bemessen, dass sie den Bedarf für die Herstellung von TDI **16a** aus Toluol **10b,** Salpetersäure **7b,** Wasserstoff **12a,** Phosgen (COCh) unter Nutzung der Einheiten **10, 12** und **16** der Vorrichtung übererfüllt.

Die für die weitere Dampf-Nutzung bereitgestellten 18 t/h des Dampfes **8a** (abs. Druck 46 bar) werden vollständig für die Deckung des Dampfbedarfs der Schwefelsäureaufkonzentrierung **11** der Einheit **10** der TDI-Prozesskette genutzt, reichen aber nicht aus. In der Einheit zur Aufkonzentrierung **11** wird von der 75 Gew.-%igen abgereicherten Schwefelsäure **10d** soviel Wasser abdestilliert, bis die Zielkonzentration von 90 Gew.-% erreicht ist.

Da kein weiterer überhitzter Dampf **8a** für die Deckung des Dampfbedarfs der Prozessschritte der Einheiten **10, 12** und **16** in der TDI-Prozesskette zur Verfügung steht, wird dieser fehlende überhitzte Wasserdampf aus einer Dampfherstellung **21** unter Einsatz von externen petrochemischen Quellen bezogen.

### Beispiel 2 (Vorrichtung gemäß Fig.2, erfindungsgemäß):

Für die in Fig.2 illustrierte erfindungsgemäße Vorrichtung zur Herstellung von TDI als organischem, aromatischem Isocyanat **16a** wird die Produktionseinheit **1** zur Bereitstellung von Salpetersäure **7b** und überhitztem Wasserdampf **8a** wie folgt konfiguriert. Die Produktionseinheit **1** produziert in einem elektrifizierten Prozess 70 t/h 68%ige Salpetersäure **7b.** Die im Beispiel 1 genutzte Dampfturbine **20** zum Antrieb der Verdichtungseinheiten **2, 3** wird durch einen mit elektrischem Strom **19a** aus regenerativer Energie betriebenen Elektro-Motor **19** mit einer Nennleistung von 6,3 MW ersetzt. Durch den Wegfall der Dampf-Turbine **20** steht die volle Menge der erzeugten 48 t/h (abs. Druck 46 bar)-Dampf **8a** für die energieeffiziente Dampf-Nutzung in der der Produktionseinheit 1 nachfolgenden Prozesskette der Einheiten **10, 12** und **16** zur Verfügung.

Die Gesamtheit der produzierten Salpetersäure **7b** (70 t/h) ist derart bemessen, dass sie den Bedarf für die Herstellung von TDI **16a** aus Toluol **10b,** Salpetersäure **7b,** Wasserstoff **12a,** Phosgen (COCh) unter Nutzung der Einheiten **10, 12** und **16** der Vorrichtung übererfüllt.

Die für die energieeffiziente Dampf-Nutzung bereitgestellten 48 t/h des Dampfes **8a** (abs. Druck 46 bar) werden zu 90% in der TDI-Prozessskette der Einheiten **10, 12** und **16** genutzt, wobei der größte Anteil davon auf die Schwefelsäure-Aufkonzentrierung **11** der Nitriereinheit **10** entfällt, und ein kleiner Anteil davon auf die Aufreinigungs- / Destillations- und Rektifikations--Vorrichtungen **14, 15, 17, 18** der Einheiten **12** und **16.**

Der verfügbare energieeffiziente Dampf **8a** aus der Salpetersäureproduktion 1 deckt in diesem erfindungsgemäßen Beispiel den gesamten verbleibenden Dampf-Bedarf der nachfolgenden TDI-Prozesskette der Einheiten **10, 12** und **16.**

## Patentansprüche

1. Vorrichtung zur Herstellung von organischem, aromatischem Isocyanat, enthaltend :
mindestens eine Produktionseinheit (1) zur Bereitstellung von Salpetersäure (7b) und Wasserdampf (8a), wobei die Produktionseinheit (1) enthält:
mindestens eine Verdichtungseinheit (2), enthaltend mindestens einen Kompressor zur Verdichtung von Sauerstoff-haltigem Gas (2a) und mindestens einen Auslass für das komprimierte, Sauerstoff-haltige Gas (2b); und
mindestens eine Bereitstellungsvorrichtung (5) zur Bereitstellung von Ammoniak; und
mindestens einen Oxidationsreaktor (6) zur Oxidation des bereitgestellten Ammoniaks im Gemisch zumindest mit Sauerstoff aus dem komprimierten, Sauerstoff-haltigen Gas (2b) zu einem Stickstoffoxid-haltigen Produktgas (6a) unter Bereitstellung von Wärme (9a);
wobei der Oxidationsreaktor (6) mindestens einen Auslass für Stickstoffoxid-haltiges Produktgas (6a) aufweist;
und
mindestens eine Verdichtungseinheit (3), enthaltend mindestens einen Kompressor zur Verdichtung zumindest des Stickstoffoxids aus dem Produktgas (6a) und mindestens einen Auslass für komprimiertes, Stickstoffoxid-haltiges Produktgas (3a);
mindestens einen Absorptionsreaktor (7) zur Umsetzung von zumindest Stickstoffoxid aus dem komprimierten Produktgas (3a) mit Wasser (7a) zu Salpetersäure (7b); und
mindestens eine Dampfeinheit (8) zur Bereitstellung von überhitztem Wasserdampf (8a) mit einer Temperatur von mindestens 200°C, wobei diese Dampfeinheit (8) mindestens einen Wärmetauscher (9) enthält, der zur Abführung von Wärme (9a) aus dem Oxidationsreaktor (6) und/oder/ aus dem aus dem Oxidationsreaktor herausgeführten Produktgases (6a), jeweils durch Übertragung der Wärme auf Wasser, zur Bereitstellung des überhitzten Wasserdampfes (8a), konfiguriert ist;
und
mindestens eine Nitriereinheit (10) zur Nitrierung von organischer, aromatischer Verbindung (10a) zumindest unter Einsatz von in besagter Produktionseinheit hergestellter Salpetersäure (7b) und von Schwefelsäure (10b) unter Erhalt von zumindest organischer, aromatischer Nitroverbindung (10c) und abgereicherter Schwefelsäure (10d), wobei diese Nitriereinheit (10) mindestens eine Einheit zur Aufkonzentrierung (11) der bei der Nitrierung anfallenden abgereicherten Schwefelsäure (10d) enthält und diese Nitriereinheit (10) mit besagter Produktionseinheit (1) zur Bereitstellung des Edukts Salpetersäure (7b) in Verbindung steht;
und
mindestens eine Hydriereinheit (12) zur Hydrierung von in der Nitriereinheit hergestellter organischer, aromatischer Nitroverbindung (10c) mit Wasserstoffgas (12a) unter Erhalt organischer, aromatischer Aminoverbindung (12b), wobei die Hydriereinheit (12) mindestens einen Hydrierreaktor (13), mindestens eine Einheit (14) zur Entwässerung von Rohprodukt und mindestens eine Destillationseinheit (15) enthält,
und wobei für die Bereitstellung der organischen, aromatischen Nitroverbindung (10c) als Edukt diese Hydriereinheit (12) mit der Nitriereinheit (10) in Verbindung steht;
und
mindestens eine Phosgeniereinheit (16) zur Phosgenierung von in der Hydriereinheit (12) hergestellter, organischer, aromatischer Aminoverbindung (12b) unter Erhalt von organischer, aromatischer Isocyanatverbindung (16a), wobei diese Phosgeniereinheit (16) mindestens eine Einheit zur Phosgenentfernung (17) und mindestens eine Destillationseinheit (18) enthält, und wobei diese Phosgeniereinheit (16) zur Bereitstellung der organischen, aromatischen Aminoverbindung (12b) mit der Hydriereinheit (12) in Verbindung steht;
**dadurch gekennzeichnet, dass** die mindestens eine Produktionseinheit (1) zur Bereitstellung von Salpetersäure (7b) und Wasserdampf (8a) zusätzlich mindestens einen Elektromotor (19) enthält, wobei die Kompressoren der besagten Verdichtungseinheiten (2, 3) der Produktionseinheit (1) mit dem mindestens einen Elektromotor (19) zum Antrieb verbunden sind.

2. Vorrichtung gemäß Anspruch 1, **dadurch gekennzeichnet, dass** die Nitriereinheit (10), Hydriereinheit (12) und Phosgeniereinheit (16) in Summe zur Nutzung von mindestens 25 % der Gesamtmenge des in der Produktionseinheit (1) herstellbaren, überhitzten Wasserdampfes (8a) ausgelegt ist und entsprechend einer vorgesehenen Wasserdampf-Nutzung mit mindestens einem Auslass für überhitztem Wasserdampf (8a) der Dampfeinheit (8) in Verbindung steht.

3. Vorrichtung gemäß Anspruch 1 oder Anspruch 2, **dadurch gekennzeichnet, dass** besagte Nitriereinheit (10) zur Nutzung von mindestens 25 % der Gesamtmenge der in der Produktionseinheit (1) bereitgestellten Salpetersäure (7b) ausgelegt ist und mit mindestens einem Auslass für Salpetersäure (7b) des Absorptionsreaktors (7) in Verbindung steht.

4. Vorrichtung gemäß einem der vorangehenden Ansprüche, **dadurch gekennzeichnet, dass** der Elektromotor (19) mit einer Quelle für elektrischen Strom aus erneuerbarer Energie (19a), insbesondere aus Windkraft, Wasserkraft oder Sonnenenergie, verbunden ist.

5. Vorrichtung gemäß einem der vorangehenden Ansprüche, **dadurch gekennzeichnet, dass** besagter Oxidationsreaktor (6) zur Bereitgestellung von mindestens 20 MW Wärmeenergie (9a), bevorzugt mindestens 30 MW Wärmeenergie (9a), ausgelegt ist.

6. Vorrichtung gemäß einem der vorangehenden Ansprüche, **dadurch gekennzeichnet, dass** besagte Vorrichtung zur Erzeugung eines Wasserdampf-Stromes dafür geeignet ist, einen besagten Wasserdampf-Strom (8a) mit einem absoluten Druck von mindestens 6 bar, bevorzugt von mindestens 11 bar, zu erzeugen.

7. Vorrichtung gemäß einem der vorangehenden Ansprüche, **dadurch gekennzeichnet, dass** überhitzter Wasserdampf (8a), der in der Produktionseinheit (1) zur Bereitstellung von Salpetersäure (7b) und Wasserdampf (8a) bereitgestellt wird, in einer Verwertungsvorrichtung genutzt werden kann, die kein Bestandteil der Produktionseinheit (1) zur Bereitstellung von Salpetersäure (7b) und Wasserdampf (8a) ist mit der Maßgabe, dass mindestens ein Auslass der Dampfeinheit (8) für überhitztem Wasserdampf in Verbindung mit der besagten Verwertungsvorrichtung steht.

8. Vorrichtung gemäß einem der vorangehenden Ansprüche, **dadurch gekennzeichnet, dass** überhitzter Wasserdampf (8a), der in der Produktionseinheit (1) zur Bereitstellung von Salpetersäure (7b) und Wasserdampf (8a) bereitgestellt wird, in mindestens einer Verwertungsvorrichtung genutzt werden kann, wobei die Verwertungsvorrichtung mindestens eine Einheit enthält, ausgewählt aus Destillationseinheit, Entwässerungseinheit, Wärmetauscher, Dampf-Turbine,
mit der Maßgabe, dass mindestens ein Auslass der Dampfeinheit (8) für überhitztem Wasserdampf (8a) in Verbindung mit der besagten Verwertungsvorrichtung steht.

9. Vorrichtung gemäß einem der Ansprüche 7 oder 8, **dadurch gekennzeichnet, dass** mindestens eine der Verwertungsvorrichtungen ein Teil einer Einheit, ausgewählt aus der Nitriereinheit (10), der Hydriereinheit (12), der Phosgeniereinheit (16) oder einer Kombination aus mindestens zwei dieser Einheiten, ist.

10. Verfahren zur Herstellung von organischem, aromatischem Isocyanat, enthaltend zumindest die folgenden Schritte:
Herstellung von Salpetersäure und überhitztem Wasserdampf mit einer Temperatur von mindestens 200°C durch zumindest folgende Schritte:
Verdichtung eines Sauerstoff-haltigen Gases zu komprimiertem, Sauerstoff-haltigem Gas unter Einsatz mindestens eines Kompressors einer Verdichtungseinheit, der zumindest durch mindestens einen Elektromotor angetrieben wird;
Vermischen von Ammoniak mit dem komprimierten, Sauerstoff-haltigen Gas und anschließender Oxidation des Ammoniaks unter Erhalt von Stickstoffoxid-haltigem Produktgas und Wärme;
Umwandlung der Wärme aus obiger Oxidation unter Einsatz mindestens eines Wärmetauschers zu einem Strom von überhitztem Wasserdampf mit einer Temperatur von mindestens 200°C;
Verdichtung des Stickstoffoxid-haltigen Produktgases aus der Oxidation unter Einsatz mindestens eines weiteren Kompressors einer weiteren Verdichtungseinheit, der durch einen Elektromotor angetrieben wird;
Umsetzung von Stickstoffoxid aus dem komprimierten Stickstoffoxid-haltigem Produktgas mit Wasser zu Salpetersäure;
Transport des überhitzten Wasserdampfes durch eine Transporteinheit zu mindestens einer Einheit zur Nutzung des Wasserdampf-Stromes;
Herstellung von organischer, aromatischer Nitroverbindung durch Umsetzung von organischer, aromatischer Verbindung mit zumindest der zuvor hergestellten Salpetersäure und mit Schwefelsäure;
Hydrierung der organischen, aromatischen Nitroverbindung mit Wasserstoffgas zu organischer, aromatischer Aminoverbindung und Aufarbeitung des Rohproduktes durch zumindest Destillation;
Phosgenierung der organischen, aromatischen Aminoverbindung mit Phosgen zu organischer, aromatischer Isocyanatverbindung und Aufarbeitung des Rohproduktes durch zumindest Destillation;
mit der Maßgabe, dass die Einheit zur Nutzung des Wasserdampf-Stromes von den zuvor genannten Einheiten zur Verdichtung von Sauerstoff-haltigem Gas und zur Verdichtung von Stickstoffoxid-haltigem Gas verschieden ist.

11. Verfahren gemäß Anspruch 10, **dadurch gekennzeichnet, dass** die besagten Verdichtungseinheiten zumindest durch mindestens einen Elektromotor angetrieben werden, der mit elektrischem Strom aus erneuerbarer Energie, insbesondere aus Windkraft, Wasserkraft oder Sonnenenergie, betrieben wird.

12. Verfahren gemäß Anspruch 10 oder Anspruch 11, **dadurch gekennzeichnet, dass** die besagten Verdichtungseinheiten zumindest durch denselben Elektromotor angetrieben werden.

13. Verfahren gemäß einem der Ansprüche 10 bis 12, **dadurch gekennzeichnet, dass** besagte bei der Oxidation bereitgestellte Wärme eine Wärmeenergie von mindestens 20 MW, bevorzugt von mindestens 30 MW besitzt.

14. Verfahren gemäß einem der Ansprüche 10 bis 13, **dadurch gekennzeichnet, dass** der besagte Wasserdampf-Strom einen absoluten Druck von mindestens 6 bar, bevorzugt von mindestens 11 bar, aufweist.

15. Verfahren gemäß einem der Ansprüche 10 bis 14, **dadurch gekennzeichnet, dass** die Einheit zur Nutzung des WasserdampfStromes mindestens eine Verwertungsvorrichtung ausgewählt aus Wärmetauscher und/oder Dampf-Turbine enthält.

16. Verfahren gemäß einem der Ansprüche 11 bis 15, **dadurch gekennzeichnet, dass** es in einer Vorrichtung gemäß einem der Ansprüche 1 bis 9 ausgeführt wird.

17. Verwendung der mindestens einen Produktionseinheit (1) für Salpetersäure (7b) und überhitztem Wasserdampf (8a) in einer Vorrichtung nach einem der Ansprüche 1 bis 9 zur Versorgung von mindestens einer Einheit, ausgewählt aus der Gruppe, die gebildet wird aus Nitriereinheit (10), Hydriereinheit (12) und Phosgeniereinheit (16), mit überhitztem Wasserdampf (8a) mit einer Temperatur von mindestens 200°C zur Abdeckung von mindestens 25% des Bedarfes dieser besagten Einheiten an Heizdampf mit einem absoluten Druck von mehr als 5 bar, insbesondere von mindestens 6 bar.
